# EUROPEAN PATENT SPECIFICATION

(11) **EP 1 641 496 B1**
(45) Date of publication and mention of the grant of the patent: **17.12.2008**
(21) Application number: 04777424.5
(22) Date of filing: 30.06.2004
(51) Int. Cl.: A61L 9/013, A61L 9/04, A61L 9/05, A61L 9/16, A61L 9/014

(54) **COMPOSITIONS AND METHODS FOR MANAGEMENT OF TOILET ODOR**
ZUSAMMENSETZUNGEN UND VERFAHREN ZUR BEKÄMPFUNG VON TOILETTENGERUCH
COMPOSITIONS ET PROCEDES POUR GERER LES ODEURS DE TOILETTES

(30) Priority: 30.06.2003 US 610111
(43) Date of publication of application: 05.04.2006
(73) Proprietor: S. C. Johnson & Son, Inc., Racine, WI 53403 (US)
(72) Inventor: REQUEJO, Luz, P., Racine, WI 53403 (US); MASTERSON, Daniel, J., Geneva, IL 60134 (US)
(74) Representative: Ruschke, Hans Edvard
(86) International application number: PCT/US2004/021264
(87) International publication number: WO 2005/004932

(56) References cited:
- EP-A- 1 250 938
- US-A- 4 968 496
- US-A1- 2002 127 137

## Description

### Field of the Invention

The present invention relates generally to compositions and methods for odor management, in particular to a composition and a process for managing toilet malodor arising from osmogenes.

### Background of the Invention

It is widely known that malodors (i.e., undesirable odors) can be controlled and in some instances eliminated by utilizing a deodorizing method such as a masking process, an absorption process, an ozone deodorizing process, or a catalytic process which uses a catalytic material such as a metal oxide or enzyme.

Masking processes control malodors by vaporizing and dispersing an aromatic liquid or solid such as a perfume into the ambient containing the malodor. Thus, masking processes modify the malodor to a more pleasant character by superimposing a dominant, but more pleasant odorant into the ambient. One problem with conventional aromatic liquids and solids is that such compounds tend to evaporate over an extended period of time which may result in the return of the malodor.

Absorption processes control malodors by employing an absorbent such as activated carbon or the like which absorbs odor components from the ambient. Thus, in this process, the level of intensity of the malodor is constantly being reduced from the ambient thereby refreshing the ambient.

The ozone deodorizing process serves to decompose odor components with ozone and in catalytic processes the odor components are modified in some fashion by the catalyst being used. In typical catalytic processes, enzymes are employed as the deodorizers.

In many home care applications, malodor control and/or elimination is achieved mainly by using either a masking process or an absorption process since ozone and catalytic processes are generally not feasible.

Some commonly employed odor absorbents employed in home care applications are formulations that are based on bleach oxidizing agents, peroxides, bactericides which kill microorganisms, cyclodextrins, and/or zinc ricinoleate.

EP-A-1 250 938 discloses a water-soluble odor absorption and deodorization composition comprising besides perfume, dye and surfactant, as its active ingredient 1 to 50 wt.% of a blend of zinc ricinoleate and a specific alkoxylated amine as well as a non-hygroscopic solvent, in particular water, optionally in mixture with a carboxylic acid, in a balancing amount of 50 to 90 wt.%. According to the disclosure in this document the zinc atoms of the used zinc ricinoleate are activated by the specific alkoxylated amine in order to be capable of binding with malodor molecules.

US-A-2002/0127 137 discloses a solid cast deodorizing composition which may include a deodorant (e.g. zinc ricinoleate) and a carrier (e.g. a polyethylene glycol, a fatty acid or a fatty acid alkoxylate) besides dyes and fragrances. This composition may be used for deodorizing a bath-room surface.

There has been a long felt need to improve management of toilet odor resulting from osmogenes, i.e. typical compounds or substances like hydrogen sulfide, sulfur dioxide, diphenyl sulfide, mercaptane, thioether, isovaleric acid, skatole, putrascine, thiophenols and ammonia which cause toilet malador, and which need has not been met until yet by deodorizing compositions and processes of the prior art.

Surprisingly, it has now been found that this long felt need can be met by a unique and specific new composition for managing toilet odor as claimed in the present application, which composition comprises besides fragrance and common additives like colorants, surfactants and absorbing agents, a specific combination of an effervescent component capable of liberating carbon dioxide in or out of liquid environments, a non-hygroscopic solvent and a compound capable of selectively reacting and/or binding with osmogenes as listed above and not the fragrance each in specific amounts. The unique composition for managing toilet odor claimed in the present application is not only novel over each of the cited references above but also comprises an inventive step in view of the cited prior art as will be explained below.

The object (problem) underlying the present invention is to provide a composition and a process for improving total management of toilet odor as disclosed in par. 1 of this application.

This object (problem) can be achieved (solved) by a novel composition and process as defined below, by using a specific combination of an effervescent component (a) capable of liberating carbon dioxide in or out of liquid environments, a specific non-hygroscopic solvent (b) in a specific amount and a compound (d) capable of selectively reacting and/or binding with "osmogenes" (malodor) which are produced before, during or after toilet use.

This specific combination of critical components (a), (b) and (d) is novel over each of the compositions disclosed in the documents discussed above and comprises an inventive step in view of the first discussed document representing the closest prior art if taken alone or in combination with the second discussed document in so far as it allows for the first time a total management of toilet odor which can now be controlled upon user's discretion since the product and the process can be used at any time before, during or after production of osmogens (malodor) during toilet use.

The unique, unexpected technical effect which can be achieved according to the present invention consists in that there is no more need for using specific alkoxylated amines (disclosed in the above discussed first document) as an activating agent for compound (d), e.g. zinc ricinoleate, to be effective. According to the present invention the efficacy of malodor control of the claimed composition which is designed to be in powder form is from the synergistic effect of carbon dioxide and foaming formation from an effervescent component (a) used according to the present invention (e.g. a carbonate salt and an organic acid) with zinc ricinoleate. Formation of CO₂ gas plus foaming action traps the osmogenes in the toilet boil to provide ample time for zinc ricinoleate to bind the osmogenes or malodor, when the composition of the present invention is activated by contacting it with water in the toilet.

The purpose of powdered formula with powdered non-hygroscopic materials, silicon dioxide and/or magnesium carbonate is to prevent pre-mature reaction of carbonate salt and organic acid. Carbon dioxide gas has to be formed in situ to serve as CO₂ blanket to prevent osmogene gas from escaping into the environment The formation of carbon dioxide also accelerates foaming of the surfactant (if present) in the composition to further enhance the trapping of osmogenes or malodor in the toilet bowl. While osmogenes are trapped by the carbon dioxide blanket in the toilet bowl, zinc ricinoleate then binds the osmogenes (malodor).

Thus, total management of toilet odot is complete because of synergistic effect of carbon dioxide generation that accelerates foaming to enhance osmogene trapping and binding power of zinc ricinoleate. Other ingredients, such as perfume, is added to provide hedonically pleasing scent to the envirnoment after the malodorous compounds are bond by the zinc ricinoleate. Carbon dioxide gas also enhances dispersion of fragrance into the environment for faster fragrancing.

Unlike the above discussed teachings of the prior art, where formulation is in solution or concentrate, a complete toilet odor control is not feasible once osmogene gases have escaped to surrounding environment. Zinc ricinoleate will no more be able to bind the malodor once it is released to the environment.

The specific combination of an effervescent component (a), a specific non-hygroscopic solvent (b) and a compound (d) capable of selectively reacting and/or binding with osmogenes under a CO₂ blanket as used in the present invention is neither disclosed in any of the above discussed documents nor made obvious by them.

The subject-matter of the present invention is according to a first aspect a composition for managing toilet odor consisting essentially of:
a) an effervescent component comprising an acid and a salt and capable of liberating carbon dioxide in or out of liquid environments;
b) a non-hygroscopic, non-aqueous solvent selected from the group consisting of diethyl phthalate, isopropyl myristate, isopropyl palmitate, dioctyl adipate and butyl stearate, said solvent being present in an amount of up to 10 % by weight of the composition for managing toilet odor;
c) a fragrance in an amount of 0.25 to 10 % by weight of the composition for managing toilet odor;
d) a compound capable of selectively reacting and/or binding with an osmogene and not the fragrance in an amount of 0.1 to 10 % by weight of the composition for managing toilet odor; and
e) optionally a colorant, a surfactant and/or an absorbing agent.

According to specific preferred embodiments of the present invention the composition as defined above comprises one or more of the following features:
the osmogene is selected from the group consisting of hydrogen sulfide, sulfur dioxide, diphenyl sulfide, mercaptane, thioether, isovaleric acid, skatole, putrascine, thiophenols and ammonia;
the compound (d) which is capable of selectively reacting and/or binding with an osmogene helps to mask toilet malodor;
the compound (d) is capable of selectively reacting with an osmogene and not the fragrance is zinc ricinoleate;
the compound (d) is capable of binding an osmogene is selected from the group consisting of activated carbon, zeolites and baking soda;
the acid in component (a) is used in an amount of 10 to 60 % by weight of the composition for managing toilet odor;
the salt in component (a) is used in an amount of 10 to 70 % by weight of the selective odor suppressant (d);
the ratio of the acid/salt component (a) to the solvent (b) is not less than 9 : 1;
in component (a) the acid is citric acid and the salt is an alkali metal carbonate;
the composition further comprises a colorant, a surfactant and/or an absorbing agent;
the compostion is in powder form and does not include an aluminum salt.

The subject-matter of the present invention is according to a second aspect a process for managing toilet odor by using a composition as defined above, the process comprising:
providing a fragrance;
providing a compound capable of selectively reacting and/or binding with an osmogene and not the fragrance;
adding the fragrance to the compound capable of selectively reacting and/or binding with an osmogene and not the fragrance to form a mixture;
adding an effervescent component comprising an acid and a salt and capable of liberating carbon dioxide in or out of liquid environments,
a non-hygroscopic, non-aqueous solvent and
optionally a colorant, a surfactant and/or an absorbing agent to the mixture; and
adding the obtained composition to a toilet:

### Detailed Description of the invention

Combination of a fragrance with a compound capable of reacting with an osmogene is an effective means of managing toilet odor. In one embodiment, the present invention comprises a fragrance and a compound capable of selectively reacting with an osmogene and not the fragrance. In a second embodiment, the present invention comprises a fragrance and a compound capable of binding an osmogene. The present invention is useful for managing odor in toilets. The composition of the present invention may or may not include aluminum salts.

According to the present invention, the fragrance is typically present in an amount of 25% to 10% by weight of the composition for managing toilet odor. Any known fragrance and/or perfume may be employed. Such fragrances include, but are not limited to, Allspice, Balsam, Bouquet, Christmas Pine, Citronella, Citrus Fresh, Citrus 7305 & 7309, Clean & Fresh, Cove,Deodorizer, Earth & Sea, Eucalyptus, Evergreen, any of the Floral series (3788, 9451, 8444, 4788,9436 & 9940), Fresh & Clean 7902 & 8003, Fresh Outdoors, Gardenia, any of the Herbal series (8916,4555, 8144 & 3719), Honeysuckle, Jasmin, any of the Lemon series (6001,6039, 8136, 9413 & 9414), any of the Odor Mask series (5211,6794, 7851, 8833, 8836, 8838, 8839, 8899 & 8899 w/s), any of the Pine series (9434, 8329 & 9435), Rose (9297 & 9298), Sandalwood, Sea Breeze, Spring Clean and Spring Rain, all available from The Good Scents Company, Atlanta, Ga.

Other fragrances and/or perfumes useful in the practice of the invention include the fragrances commonly used in the household and industrial cleaning and sanitizing industry. These fragrances may be found in the catalogue Flavors & Fragrances, and are available from the Aldrich Chemical Company, Inc., Milwaukee, Wisconsin. Those of particular interest are Alpha Pinene, Alpha Terpineol, Beta Pinene, Cedar Leaf, Citral, CitronellalW23070-7, Coumarin,Diethylphthalate, Eucolyptol, Eugenol, Heptyl Isobutyrate,Trans-2-Hexene-Diol, Isobornyl and 3,5, 5-Trimehtylhexanal.

The compound capable of selectively reacting with an osmogene and not the fragrance helps manage toilet odor while not interfering with the fragrance. In certain embodiments, the compound capable of selectively reacting with the osmogene binds with the osmogene to manage its malodorous effect. In other embodiments, the compound capable of selectively reacting with the osmogene helps to mask toilet malodor. The compound may be present in an amount of 0.1 % to 10% by weight of the composition for managing toilet odor. In one embodiment, the compound is zinc ricinoleate, though other suitable compounds known to those of skill in the art may also be employed.

The compound capable of binding an osmogene also helps to manage toilet odor. This compound may also be present in an amount of 0.1 % to 10% by weight. Examples include, but are not limited to, activated carbon, zeolites and baking soda.

The term osmogene is defined broadly herein to mean any compound or substance that causes toilet malodor. Typical examples include, but are not limited to, hydrogen sulfide, sulfur dioxide, diphenyl sulfide, mercaptane, thioether, isovaleric acid, skatole, putrascine, thiophenols and ammonia.

As previously mentioned, the composition of the present invention is capable of being inserted into all toilets, either before, during or after use, to manage and/or sanitize odors. This includes toilets found in all settings with and without water holding tanks. The invention may also be used to sanitize or control odor in waste water systems.

In other aspects of the present invention, the composition for managing toilet odor may optionally include other components.

For example, in one embodiment of the present invention, the composition for managing toilet odor includes a composition capable of effervescence. The term "effervescence, "as defined herein, means forming bubbles in liquid environments and may also be considered any product capable of liberating carbon dioxide in or out of liquid environments.

In certain embodiments, the presence of bubbles results from the formation of carbon dioxide, which is also helpful in the management of toilet odor. That is, some malodors have high molecular weights and therefore cannot rise to the surface of an aqueous environment to escape into the atmosphere before carbon dioxide. Thus, in embodiments of the present invention that emit carbon dioxide, odors with high molecular weights are suppressed.

The production of carbon dioxide may be accomplished through a mixture of at least one acid and at least one salt, which when added to a liquid such as water, results in a chemical reaction that liberates carbon dioxide. In one aspect, both the acid and the salt may be in anhydrous form.

Examples of acids suitable for use in illustrative embodiments include, but are not limited to, tartaric acid, citric acid, fumaric acid, adipic acid, malic acid, oxalic acid, or sulfamic acid, either alone or in combination typically, the acid citric acid or a combination of citric acid and tartaric acid.

Examples of salts suitable for use in illustrative embodiments include, but are not limited to, the alkali metal salts. Sodium carbonate, calcium carbonate, magnesium carbonate, ammonium carbonate, potassium carbonate, sodium bicarbonate, and calcium bicarbonate may all be employed.

In other embodiments, the selection of specific acids and/or salts and their proportions depends, at least in part, upon the requirements for the amount of carbon dioxide release. In some embodiments, the acid may be added in an amount of 10% to 60% by weight of the composition for managing toilet odor, while the salt may be added in an amount of 10% to 70% by weight of the selective odor suppressant.

In other embodiments, compositions according to the present invention may include a solvent, which in select embodiments, may be added to in an amount of up to 10% by weight of the composition for managing toilet odor. The ratio of the acid/salt component to solvent should not, however, be less than 9: 1. If the ratio of the acid/salt component to the solvent falls below 9: 1, the final formulation may form a sticky mixture or slurry.

Often, but not always, the solvent is contained within the fragrance. As those of skill will appreciate, fragrances typically comprise highly concentrated solid ingredients. The presence of the solvent may be necessary to dissolve, disperse or mix these solid ingredients to make the fragrance homogenous throughout. Since fragrance manufacturers often incorporate solvents directly into their fragrances, coordinating solvent selection with the fragrance manufacturer may be necessary.

The solvent may be non-hygroscopic, defined herein as not taking up moisture from the environment. Suitable non-hygroscopic solvents include diethyl phthalate, isopropyl myristate, isopropyl palmitate and at least some species of ester solvents, such as dioctyl adipate and butyl stearate. Since neither the effervescent component nor the non-hygroscopic solvent absorb moisture, in these embodiments, the formation of bubbles is substantially avoided until the composition for managing toilet odor is contacted by a liquid, such as water. That is, moisture from the environment does not precipitate a premature reaction between the acid and salt of the present invention before these chemical constituents come into contact with liquids.

In other embodiments, the non-hygroscopic solvent may be non-aqueous. With respect to the non-aqueous aspect, the absence of water from the selective odor suppressant substantially avoids the formation of bubbles until the effervescent is contacted by a liquid. Regarding the absence of polarity, this feature avoids dissociation of the salt, which under certain circumstances, may trigger a pre-mature reaction between the acid and carbonate or bicarbonate components of the effervescent component.

The composition for managing toilet odor may further include a colorant. The colorant may be oil- or water-soluble, and typically is an anhydrous powder dye. The amount of colorant to be used may depend on the color intensity desired and the cost of the dye, and may be added at levels up to about 2.0% by weight of the composition for managing toilet odor. When the composition for managing toilet odor includes a colorant, but not an effervescent component, a small amount of water for dissolving the colorant may be added. The water is present in an amount of no more than about 1.0% by weight of the composition for managing toilet odor.

The choice of the colorant will depend largely on the color desired for the water into which the effervescent is to be dispensed. Examples of suitable water- soluble colorants include, but are not limited to, Acid Blue #9, Basacid Blue NB 755^{®}, FD & C Yellow #5, FD & C Red #33, and D & C Green #8. Oil-soluble colorants include, but are not limited to, Nitro Fast Red A 4B^{®}, Solvent Yellow 72 and Sandoplast Green G^{®}.

In addition, the composition for managing toilet odor may further comprise a surfactant. A surfactant is any substance capable of reducing surface tension, no matter how slight, between phases. Similar to the colorant, the surfactant is typically added in anhydrous form. In some embodiments, the surfactant may be added at levels ranging from 0.5% to 10% by weight of the composition for managing toilet odor. In other embodiments, the surfactant imparts a detergent effect to the present invention.

Surfactants useful with the present invention include anionic, non-ionic, cationic, amphoteric and zwitterionic surfactants. Anionic surfactants are particularly useful, since such surfactants are capable of forming a thick foam or lather during liberation of carbon dioxide by the effervescent component.

Examples of suitable surfactants include, but are not limited to, sodium lauryl sulfonate, sodium alpha olefin sulfonate, alkyl benzene sulfonate, sodium dodecyl benzene sulfonate and cocoyl glutamic acid. Other types of surfactants include alkyl benzenesulfonates, alkyl ether sulfates, paraffin, sulfonates, olefin sulfonates, amine oxides, alkyl betaines and the like, which are known in the art. Commercial sources of such surfactants may be found in McCutcheon's Emulsifiers and Detergents, North American Edition, 1987, McCutcheon Division (MC Publishing Company).

An absorbing or flowing agent may also be added to the composition of the present invention, in an amount of 0.1 % to 15% by weight. The absorbing or flowing agent is useful for preventing the premature reaction between the acid and carbonate or bicarbonate components of the composition, and typically is a compound comprising silica (silicon dioxide). Generally, magnesium carbonate is employed. Other examples of absorbing agents, include but are not limited to, amorphous silica, foamed silica and synthetic silica.

The various forms of the composition for managing toilet odor may be contained in a reagent vessel. A reagent vessel is capable of containing or holding the composition of the present invention. For example, the reagent vessel may be a sealed pouch, dissolvable in water. In such embodiments, the reagent vessel may be constructed from a permeable filter paper-like material (e. g. the material used in tea bags). This type of material permits the components to leave the reagent vessel after insertion into the toilet so that the components may react in the toilet water.

The reagent vessel may also be constructed of foil, plastic or any other type of material that will hold the composition of the present invention before insertion into a toilet. Such materials include, but are not limited to, polyethylene, polypropylene, polystyrene and polyethylene-terephtalate. In these embodiments, the reagent vessel may be discarded prior to addition of the composition of the present invention into the toilet. For instance, the various forms of the composition may be packaged in a single use package, which may be carried in one's purse or pocket. When using a public restroom or a friend's bathroom, the user may open the package containing the composition of the present invention, and dispense its contents into the toilet.

When the composition of the present invention includes an effervescent component and is contained within a reagent vessel, it is particularly useful to substantially avoid bubbling by the effervescent component until it contacts a liquid.

More specifically, when bubbling results in carbon dioxide formation, the presence of carbon dioxide gas may exert pressure on the reagent vessel causing it to explode and/or prematurely release its contents before being inserted into a toilet. Under these circumstances, the entire composition for managing toilet odor may be rendered unusable and/or potentially injure the user.

In still other embodiments, a dispensing apparatus for delivering the composition of the present invention into a toilet may be employed. The dispensing apparatus may, for example, be attached to any solid surface on or above a toilet and may comprise a container connected to a tube or other device for delivering the composition from the container into the toilet bowl. The dispensing apparatus may be operated by depressing a button or any other suitable means that will dispense appropriate amounts of the composition for controlling toilet odor.

Additionally, the composition for managing toilet odor may include filler material to make the formula more affordable. Examples of suitable fillers include, but are not limited to, sodium sulfate and sodium chloride.

The various forms of the composition for managing toilet odor may be contained in a reagent vessel. A reagent vessel is capable of containing or holding the composition of the present invention. For example, the reagent vessel may be a sealed pouch, dissolvable in water. In such embodiments, the reagent vessel may be constructed from a permeable filter paper-like material (e. g. the material used in tea bags). This type of material permits the components to leave the reagent vessel after insertion into the toilet so that the components may react in the toilet water.

The reagent vessel may also be constructed of foil, plastic or any other type of material that will hold the composition of the present invention before insertion into a toilet. Such materials include, but are not limited to, polyethylene, polypropylene, and polyethylene-terephtalate. In these embodiments, the reagent vessel may be discarded prior to addition of the composition of the present invention into the toilet. For instance, the various forms of the composition may be packaged in a single use package, which may be carried in one's purse or pocket. When using a public restroom or a friend's bathroom, the user may open the package containing the composition of the present invention, and dispense its contents into the toilet.

When the composition of the present invention includes an effervescent component and is contained within a reagent vessel, it is particularly useful to substantially avoid bubbling by the effervescent component until it contacts a liquid.

More specifically, when bubbling results in carbon dioxide formation, the presence of carbon dioxide gas may exert pressure on the reagent vessel causing it to explode and/or prematurely release its contents before being inserted into a toilet. Under these circumstances, the entire composition for managing toilet odor may be rendered unusable and/or potentially injure the user.

In still other embodiments, a dispensing apparatus for delivering the composition of the present invention into a toilet may be employed. The dispensing apparatus may, for example, be attached to any solid surface on or above a toilet and may comprise a container connected to a tube or other device for delivering the composition from the container into the toilet bowl or tank. The dispensing apparatus may be operated by depressing a button or any other suitable means that will dispense appropriate amounts of the composition for controlling toilet odor.

In other aspects, the present invention involves methods of making the composition for managing toilet odor described herein. In illustrative embodiments, compositions according to the present invention may be prepared in a V-blender or a ribbon blender.

If a V-blender is employed, in one embodiment, a fragrance and a compound capable of binding an osmogene or a compound capable of selectively reacting with an osmogene and not the fragrance are mixed together in the blender. Additional components, such as filler materials, may also be added. In embodiments that comprise an effervescent component, a salt and citric acid are first added to the V- blender. Mixing of the compounds for approximately fifteen minutes occurs, followed by addition of the fragrance and further mixing. Finally, the compounds that react with osmogenes are added, followed by mixing until substantially homogeneous.

Optionally, additional materials may be added to any of the formulations. For example, a surfactant, a flowing agent, a solvent for the fragrance or a dye may be incorporated into the process. In most cases, however, the compound capable of reacting with the osmogene should be added last.

A ribbon blender may also be employed. This processing method is faster and more efficient. In embodiments using a ribbon blender, the chemical constituents may be added simultaneously, followed by mixing.

Regardless of the type of blender employed, external conditions, such as temperature and humidity, should be monitored throughout the process of making the composition of the present invention. Ideally, manufacture of the composition is carried out at temperatures between 18 C and 30 C and at lower relative humidities, up to a maximum of 40% for example, with a preferred relative humidity below 25%.

Typically, the above-described method of making yields the composition of the present invention in powder form. The composition may, however, be supplied in various other forms, such as tablet form, block form, cake form, capsule or gel form, and any other form known to those of skill in the art. To make tablet, block or cake forms, the powder form may be compressed by methods known to those of skill in the art. Size and hardness are dependent on the mould size and pressure used during the compression process. If a liquid form is desired, in some cases it is necessary to employ a dual bottle with two compartments to separate the effervescent components of the invention.

Variations, modifications and other implementations of what is described herein will occur to those of ordinary skill in the art without departing from the spirit and scope of the invention. Accordingly, the invention is in no way limited by the preceding illustrative description.

### Industrial Applicability

The present invention has applicability in the management of odor in toilets. This includes toilets found in all settings with and without water holding tanks. The present invention may also be used to sanitize or control odor in waste water systems.

## Claims

1. A composition for managing toilet odor consisting essentially of:
a) an effervescent component comprising an acid and a salt and capable of liberating carbon dioxide in or out of liquid environments;
b) a non-hygroscopic, non-aqueous solvent selected from the group consisting of diethyl phthalate, isopropyl myristate, isopropyl palmitate, dioctyl adipate and butyl stearate, said solvent being present in an amount of up to 10 % by weight of the composition for managing toilet odor;
c) a fragrance in an amount of 0.25 to 10 % by weight of the composition for managing toilet odor;
d) a compound capable of selectively reacting and/or binding with an osmogene and not the fragrance in an amount of 0.1 to 10 % by weight of the composition for managing toilet odor, and
e) optionally a colorant, a surfactant and/or an absorbing agent.

2. The composition of claim 1, wherein the osmogene is selected from the group consisting of hydrogen sulfide, sulfur dioxide, diphenyl sulfide, mercaptane, thioether, isovaleric acid, skatole, putrascine, thiophenols and ammonia.

3. The composition of any of claims 1 to 2, wherein the compound (d) capable of selectively reacting and/or binding with an osmogene helps to mask toilet malodor.

4. The composition of any of claims 1 to 3, wherein the compound (d) capable of selectively reacting with an osmogene and not the fragrance is zinc ricinoleate.

5. The composition of any of claims 1 to 3, wherein the compound (d) capable of binding an osmogene is selected from the group consisting of activated carbon, zeolites and baking soda.

6. The composition of any of claims 1 to 5, wherein the acid in component (a) is used in an amount of 10 to 60 % by weight of the composition for managing toilet odor.

7. The composition of any of claims 1 to 6, wherein the salt in component (a) is used in an amount of 10 to 70 % by weight of the selective odor suppressant (d).

8. The composition of any of claims 1 to 7, wherein the ratio of the acid/salt component (a) to the solvent (b) is not less than 9 : 1.

9. The composition of any of claims 1 to 8, wherein in component (a) the acid is citric acid and the salt is an alkali metal carbonate.

10. The composition of any of claims 1 to 9, further comprising a colorant, a surfactant and/or an absorbing agent.

11. The composition of any of claims 1 to 10, wherein the composition does not include an aluminum salt.

12. The composition of any of claims 1 to 11, wherein the compostion is in powder form.

13. A process for managing toilet odor by using a composition of any of claims 1 to 12, the process comprising:
providing a fragrance;
providing a compound capable of selectively reacting and/or binding with an osmogene and not the fragrance;
adding the fragrance to the compound capable of selectively reacting and/or binding with an osmogene and not the fragrance to form a mixture;
adding an effervescent component comprising an acid and a salt and capable of liberating carbon dioxide in or out of liquid environments,
a non-hygroscopic, non-aqueous solvent and
optionally a colorant, a surfactant and/or an absorbing agent to the mixture; and
adding the obtained composition to a toilet.

## Patentansprüche

1. Mittel zum Bekämpfen von Toilettengerüchen, im Wesentlichen bestehend aus:
a) einer Aufschäumkomponente, die eine Säure und ein Salz aufweist und Kohlenstoffdioxid in oder aus flüssigem Milieu freisetzen kann;
b) einem nicht hygroskopischen und nicht wässrigen Lösungsmittel, das gewählt ist aus der Gruppe Diethylphthalat, Isopropylmyristat, Isopropylpalmitat, Dioctyladipat und Butylstearat, wobei das Lösungsmittel in einer Menge bis zu 10 Gew.% des Gewichts des Mittels zum Bekämpfen von Toilettengerüchen vorliegt;
c) einem Duftstoff in einer Menge von 0,25 Gew.-% bis 10 Gew.-% des Mittels zur Beseitigung des Mittels zum Bekämpfen von Toilettengrüchen;
d) einer Verbindung, die mit einem Osmogen, nicht aber dem Duftstoff selektiv reagieren und/oder sich an es bzw. ihn binden kann; und
e) wahlweise einem Färbe-, einem grenzflächenaktiven und / oder einem Absorptionsmittel.

2. Mittel nach Anspruch 1, bei dem das Osmogen gewählt ist aus der Gruppe Wasserstoffsulfid, Schwefeldioxid, Diphenylsulfid, Mercaptan, Thioäther, Isovaleriansäure, Skatol, Putrascin, den Thiophenolen und Ammoniak.

3. Mittel nach Anspruch 1 oder 2, bei dem die Verbindung (d), die wahlweise mit einem Osmogen, nicht aber dem Duftstoff reagieren und / oder sich an es bzw. ihn binden kann, das Überdecken übler Toilettengerüche unterstützt.

4. Mittel nach einem der Ansprüche 1 bis 3, bei dem die Verbindung (d), die wahlweise mit einem Osmogen, nicht aber dem Duftstoff reagieren und/oder sich an es bzw. ihn binden kann, Zinkrizinoleat ist.

5. Mittel nach einem der Ansprüche 1 bis 3, bei dem die Verbindung (d), die wahlweise mit einem Osmogen reagieren und / oder sich an es binden kann, gewählt ist aus der Gruppe Aktivkohle, Zeolite und Natriumbicarbonat (Backnatron).

6. Mittel nach einem der Ansprüche 1 bis 5, bei dem die Säure in der Komponente (a) in einer Menge von 10 Gew.-% bis 60 Gew.-% des Mittels zum Bekämpfen von Toilettengerüchen verwendet wird.

7. Mittel nach einem der Ansprüche 1 bis 6, bei dem das Salz in der Komponente (a) in einer Menge von 10 Gew.-% bis 70 Gew.-% des wahlweise anzuwendenden Geruchsminderers (d) eingesetzt wird.

8. Mittel nach einem der Ansprüche 1 bis 7, bei dem das Verhältnis der Säure/Salz-Komponente (a) zum Lösungsmittel (b) nicht geringer ist als 9:1.

9. Mittel nach einem der Ansprüche 1 bis 8, bei dem in der Komponente (a) die Säure Zitronensäure und das Salz ein Alkalimetallcarbonat ist.

10. Mittel nach einem der Ansprüche 1 bis 9 weiterhin mit einem Färbe-, einem grenzflächenaktiven und/oder einem Absorptionsmittel.

11. Mittel nach einem der Ansprüche 1 bis 10, das kein Aluminiumsalz enthält.

12. Mittel nach einem der Ansprüche 1 bis 11, das als Pulver vorliegt.

13. Verfahren zum Bekämpfen von Toilettengerüchen durch Anwenden eines Mittels nach einem der Ansprüche 1 bis 12 mit folgenden Schritten:
Bereitstellen eines Duftstoffs;
Bereitstellen einer Verbindung, die wahlweise mit einem Osmogen, nicht dem Farbstoff reagieren und / oder es binden kann;
Zugeben des Duftstoffs zu der Verbindung, die wahlweise mit einem Osmogen, nicht dem Farbstoff reagieren und/oder es binden kann; sowie
Zugeben einer Aufschäumkomponente, die eine Säure und ein Salz aufweist und Kohlenstoffdioxid in oder aus flüssigem Milieu freisetzen kann,
eines nicht hygroskopischen, nicht wässrigen Lösungsmittels und
wahlweise eines Färbe-, eines grenzflächenaktiven und / oder eines Absorptionsmittels zur Mischung sowie
Einbringen der erhaltenen Zusammensetzung in eine Toilette.

## Revendications

1. Composition pour la maîtrise des odeurs dans les toilettes essentiellement constituée:
a) d'un constituant effervescent comprenant un acide et un sel et capable de libérer du dioxyde de carbone dans ou à l'extérieur d'environnements liquides ;
b) d'un solvant non-aqueux, non-hygroscope choisi parmi le phtalate de diéthyle, le myristate d'isopropyle, le palmitate d'isopropyle, l'adipate de dioctyle et le stéarate de butyle, ledit solvant étant présent dans une quantité allant jusqu'à 10 % en poids de la composition pour la maîtrise des odeurs dans les toilettes ;
c) d'un parfum dans une quantité de 0,25 à 10 % en poids de la composition pour la maîtrise des odeurs dans les toilettes ;
d) d'un composé capable de réagir et/ou de se lier sélectivement avec un osmogène et non pas avec le parfum dans une quantité de 0,1 à 10 % en poids de la composition pour la maîtrise des odeurs dans les toilettes, et
e) facultativement d'un colorant, d'un tensioactif et/ou d'un agent absorbant.

2. Composition selon la revendication 1, dans laquelle l'osmogène est choisi parmi le sulfure d'hydrogène, le dioxyde de soufre, le sulfure de diphényle, un mercaptan, un thioéther, l'acide isovalérique, le scatale, la putrescine, des thiophénols et l'ammoniac.

3. Composition selon l'une quelconque des revendications 1 à 2, dans laquelle le composé (d) capable de réagir et/ou de se lier sélectivement avec un osmogène aide à masquer les odeurs nauséabondes dans les toilettes.

4. Composition selon l'une quelconque des revendications 1 à 3, dans laquelle le composé (d) capable de réagir sélectivement avec un osmogène et non pas avec le parfum est le ricinoléate de zinc.

5. Composition selon l'une quelconque des revendications 1 à 3, dans laquelle le composé (d) capable de lier un osmogène est choisi parmi le charbon actif, des zéolites et la levure chimique.

6. Composition selon l'une quelconque des revendications 1 à 5, dans laquelle l'acide dans le constituant (a) est utilisé dans une quantité de 10 à 60 % en poids de la composition pour la maîtrise des odeurs dans les toilettes.

7. Composition selon l'une quelconque des revendications 1 à 6, dans laquelle le sel dans le constituant (a) est utilisé dans une quantité de 10 à 70 % en poids de l'agent sélectif supprimant une odeur (d).

8. Composition selon l'une quelconque des revendications 1 à 7, dans laquelle le rapport du constituant acide/sel (a) au solvant (b) n'est pas inférieur à 9 : 1.

9. Composition selon l'une quelconque des revendications 1 à 8, dans laquelle dans le constituant (a) l'acide est l'acide citrique et le sel est un carbonate de métal alcalin.

10. Composition selon l'une quelconque des revendications 1 à 9 comprenant de plus un colorant, un tensioactif et/ou un agent absorbant.

11. Composition selon l'une quelconque des revendications 1 à 10, dans laquelle la composition ne comprend pas un sel d'aluminium.

12. Composition selon l'une quelconque des revendications 1 à 11, dans laquelle la composition est dans une forme de poudre.

13. Procédé pour la maîtrise des odeurs dans les toilettes utilisant une composition selon l'une quelconque des revendications 1 à 12, le procédé comprenant :
la fourniture d'un parfum ;
la fourniture d'un composé capable de réagir et/ou de se lier sélectivement avec un osmogène et non pas avec le parfum ;
l'addition du parfum au composé capable de réagir et/ou de se lier sélectivement avec un osmogène et non pas avec le parfum pour former un mélange ;
l'addition d'un constituant effervescent comprenant un acide et un sel et capable de libérer du dioxyde de carbone dans ou à l'extérieur d'environnements liquides,
d'un solvant non-hygroscope, non-aqueux et
facultativement d'un colorant, d'un tensioactif et/ou d'un agent absorbant au mélange ; et
l'addition de la composition obtenue à des toilettes.
